# FASCICULE DE BREVET EUROPEEN

(11) **EP 0 716 706 B1**
(45) Date de publication et mention de la délivrance du brevet: **18.10.2006**
(21) Numéro de dépôt: 94925521.0
(22) Date de dépôt: 19.08.1994
(51) Int. Cl.: C12N 15/82, C12N 5/10

(54) **PROCEDE DE PRODUCTION DE PLANTES TRANSGENIQUES, ENTIEREMENT TRANSFORMEES EN GENERATION T O?, A PARTIR DE MERISTEMES**
VERFAHREN ZUR HERSTELLUNG VON IN AUS MERISTEN DERIVIERTE TO-GENERATION VÖLLIG TRANSFORMIERTEN TRANSGENEN PFLANZEN
METHOD OF PRODUCTION OF TRANSGENIC PLANTS WHOLLY TRANSFORMED INTO T O? GENERATION, FROM MERISTEMS

(30) Priorité: 30.08.1993 FR 9310368
(43) Date de publication de la demande: 19.06.1996
(73) Titulaire: Biogemma, 75001 Paris (FR)
(72) Inventeur: KNITTEL, Nathalie, Clifton Hill, VIC 3068 (AU); LENEE, Philippe, F-63110 Beaumont (FR)
(74) Mandataire: Almond-Martin, Carol
(86) Numéro de dépôt international: PCT/FR1994/001017
(87) Numéro de publication internationale: WO 1995/006741

(56) Documents cités:
- EP-A- 0 301 749
- EP-A- 0 400 553
- EP-A- 0 424 047
- WO-A-89/12102
- WO-A-92/15675
- GB-A- 2 211 204
- BIOTECHNOLOGY, vol.11, no.5, Mai 1993, NEW YORK US pages 596 - 598 MCCABE, D.E., ET AL. 'Transformation of elite cotton cultivars via particle bombardment'
- PROCEEDINGS OF THE NATIONAL ACADEMY OF SCIENCES OF USA, vol.86, Octobre 1989, WASHINGTON US pages 7500 - 7504 CHRISTOU, P., ET AL. 'Inheritance and expression of foreign genes in transgenic soybean plants'
- TIBTECH, vol.8, no.6, Juin 1990 pages 145 - 151 CHRISTOU, P., ET AL. 'Soybean genetic engineering - commercial production of transgenic plants'
- BIOTECHNOLOGY, vol.6, no.8, Août 1988, NEW YORK US pages 923 - 926 MCCABE, D.E., ET AL. 'Stable genetic transformation of soybean (Glycine max) by particle acceleration'
- PLANT MOLECULAR BIOLOGY., vol.18, 1992, DORDRECHT, THE NETHERLANDS. pages 301 - 313 BIDNEY, D., ET AL. 'Microprojectile bombardment of plant tissues increases transformation frequency by Agrobacterium' cité dans la demande
- PLANT CELL REPORTS, vol.12, no.3, Janvier 1993 pages 165 - 169 RUSSELL, D.R., ET AL. 'Stable transformation of Phaseolus vulgaris via electric-discharge mediated particle acceleration'
- PLANT CELL REPORTS, vol.9, 1990 pages 55 - 60 SCHRAMMEIJER, B., ET AL. 'Meristem transformation of sunflower via Agrobacterium'

## Description

La présente invention concerne un procédé de production de plantes transgéniques à partir de méristèmes, lesdites plantes étant entièrement transformées en génération T₀.

Les techniques de génie génétique sont, maintenant, couramment appliquées dans l'amélioration d'espèces végétales. Ces techniques permettent l'introduction de caractères nouveaux difficiles ou impossible à introduire par des techniques classiques. Malgré l'évolution de ces techniques, il existe néanmoins certaines espèces qui se prêtent difficilement à la transformation ou qui ne peuvent pas être régénérées à partir d'explants différenciés, comme des cotylédons ou des feuilles. Pour ces espèces (par exemple le tournesol, le coton, le pois, le haricot, le soja, etc...), il a été démontré que ces difficultés pouvaient, en partie, être surmontées en utilisant comme explant pour la transformation, un explant méristématique, par exemple un méristème apical.

L'utilisation de méristèmes permet la régénération de plantes fertiles sans étape de callogénèse. La technique de régénération à partir de méristèmes s'applique à un grand nombre d'espèces différentes et, au sein d'une même espèce, à un grand nombre de génotypes. Grâce à cette technique, de nombreuses espèces récalcitrantes ont pu être transformées et régénérées (voir par exemple : Bidney et al, Plant Molecular Biol. 18, 301-313, 1992 ; Bidney et al, Proc. 13e Int. Sunflower Conf. Vol II, Pisa Sept. 1992 ; Schrammeijer et al, Plant Cell Rep. 9 : 55-60, 1990 ; Christou et al, The Plant Journal, 2(3), 283-290, 1992 ; Gambley et al, Plant Cell Rep. 12 : 343-346, 1993 ; Russel et al, Plant Cell Rep. 12 : : 165-169, 1993).

Cette méthode présente néanmoins certains inconvénients. La fréquence de transformation est extrêmement faible et les plantes régénérées sont presque exclusivement chimériques, c'est-à-dire certains tissus sont transformés tandis que d'autres ne le sont pas. Cette caractéristique vient du fait que le méristème produit le matériel cellulaire à l'origine de tous les tissus des divers organes de la plante (tige, racines, feuilles). Or, quelque soit la technique employée, l'opération de transformation, ne conduit qu'à la transformation d'un nombre restreint de cellules au sein de l'explant, ces cellules étant réparties de manière alléatoire. Le méristème n'est jamais entièrement transformé à la suite d'une telle manipulation. Les plantes régénérées à partir d'un méristème ayant subi une étape de transformation sont donc chimériques. Des plantes entièrement transgéniques ne sont obtenues que dans la descendance et cela, à condition que les cellules de la lignée germinale aient été atteintes par la transformation. Ce genre de procédé est décrit par exemple dans : Bidney et al, Plant Molecular Biol. 18, 301-313, 1992 ; Schrammeijer et al, Plant Cell Rep. 9 : 55-60, 1990.

Les inconvénients présentés par l'obtention de plantes chimériques sont reconnus dans l'art, mais aucune solution n'a pu être apportée. Une système de marquage permettant de reconnaître, parmi les plantes chimériques T₀, celles qui avaient subi une transformation de la lignée germinale et qui étaient donc susceptibles de transmettre le nouveau caractère à leur descendance, a été décrit (Christou et al, The Plant Journal, 2(3), 283-290, 1992). Cette méthode facilite l'obtention, dans la génération suivante, de plantes entièrement transformées, mais les plantes produites en génération T₀ sont toujours chimériques.

A ce jour, il n'existe pas de méthode permettant de produire, de manière systématique et prévisible, des plantes transgéniques entièrement transformées en génération T₀ à partir d'explants méristématiques.

La présente invention résoud ce problème technique. L'invention se base sur la mise au point, par les inventeurs, d'une méthode permettant d'enrichir les méristèmes en cellules transformées, pour aboutir à des méristèmes entièrement transformés, c'est-à-dire des méristèmes dont toutes les cellules sont transformées selon l'invention, la régénération de plantes s'effectue ensuite exclusivement à partir de ces explants entièrement transformés, ce qui garantit le caractère transgénique des plantes obtenues. Les inventeurs ont également démontré que, dans certaines conditions, la néoformation de méristèmes et de bourgeons foliaires néoformés sur les feuilles d'explants issus de méristèmes peut être induite. L'existence de ces bourgeons foliaires néoformés, contenant des méristèmes foliaires néoformés, n'a jamais été décrite dans la littérature. Le procédé de l'invention permet également l'obtention de ces méristèmes foliaires néoformés dans un état entièrement transformé. Ils constituent alors un excellent matériel cellulaire pour la régénération de plantes entièrement transformées en T₀.

En termes généraux, l'invention fournit donc une méthode de production de méristèmes entièrement transformés, et une méthode permettant de régénérer exclusivement à partir de ces explants, des plantes entièrement transgéniques en T0.

La présente demande décrit un procédé de production de plantes transgéniques, entièrement transformées en génération T₀, comprenant :
a) une étape de transformation génétique d'un explant méristématique, et
b) une étape de culture sélective permettant le développement spécifique, parmi toutes les cellules transformées, de celles qui sont à l'origine des méristèmes secondaires et/ou de celles capables de donner lieu à des méristèmes foliaires néoformés;
c) la régénération, à partir du matériel cellulaire obtenu lors de l'étape ii), de plantes transgéniques.

Dans le contexte de la présente invention, le terme "explant méristématique" signifie un explant constitué essentiellement ou exclusivement de tissu méristématique ou de tissu susceptible de devenir, au cours de son développement, méristématique. Selon l'invention, ce terme englobe notamment :
- un méristème apical (également connu dans l'art comme "méristème primaire"), particulièrement un méristème apical caulinaire, c'est-à-dire à l'origine de la tige ;
- un bourgeon foliaire néoformé ;
- une partie d'une jeune feuille capable de donner lieu à des bourgeons foliaires néoformés.

Le terme "bourgeon foliaire néoformé" signifie un bourgeon porté sur l'épiderme supérieur d'une feuille. Il contient un méristème foliaire néoformé. Son développement est induit de manière "artificielle" par une série de conditions de culture précises.

La nature de ces différents explants sera décrite en détail plus loin.

Le terme "méristème secondaire" signifie un méristème né d'un méristème primaire. En particulier, ce terme vise, dans le contexte de l'invention, les méristèmes axillaires, situés à l'aisselle des feuilles et responsables de la construction des rameaux latéraux de la plante.

Le terme "bourgeon axillaire" signifie un bourgeon situé à l'aisselle d'une feuille, contenant un méristème secondaire.

Le terme "méristème foliaire néoformé" signifie un méristème contenu dans un bourgeon foliaire néoformé. La formation de ces méristèmes est provoquée "artificiellement" par des conditions de culture qui seront définies plus loin.

Au niveau cellulaire, les méristèmes secondaires et les méristèmes foliaires néoformés ont une organisation structurale et un fonctionnement identiques à ceux du méristème principal, mais sont de tailles plus petites. Par ailleurs, les méristèmes foliaires néoformés sont plus petits que les méristèmes secondaires.

Les travaux conduisant à l'élaboration de la présente invention sont basés sur les principes morphogénétiques suivants. Le méristème apical contient, à l'aisselle des Primordia foliaires, des cellules qui, de manière prédéterminée, donneront naissance, par multiplication cellulaire, à des méristèmes secondaires. Il se peut, lors d'une opération de transformation génétique, que ces cellules "pré-programmées" soient transformées. Les méristèmes secondaires issus de la multiplication de ces cellules seront composés exclusivement de cellules transformées. Il en est de même pour les cellules qui, dans des conditions de culture favorables, donneront naissance à des méristèmes foliaires néoformés. Par conséquent, les plantes régénérées à partir de ces méristèmes secondaires transformés et à partir des méristèmes foliaires néoformés transformés seront entièrement transgéniques.

Les inventeurs ont mis au point un système de culture sélectif favorisant le développement, parmi les cellules transformées, de celles qui sont à l'origine des méristèmes secondaires et des méristèmes foliaires néoformés. Les autres cellules sont éliminées.

De préférence, la culture sélective comprend les étapes suivantes :
i) la mise en culture, sur milieu sélectif, de l'explant méristématique ayant subi l'étape de transformation;
ii) le prélèvement des bourgeons axillaires transformés et éventuellement des bourgeons foliaires néoformés transformés, obtenus au cours de l'étape i);
iii) la mise en culture sur milieu sélectif des bourgeons transformés obtenus selon ii);
iv) répétition au moins une fois des étapes ii) et iii).

Plus particulièrement, l'invention vise un procédé de production de plantes transgéniques, entièrement transformées en génération T₀, comprenant :
a. une étape de transformation génétique d'un explant méristématique ;
b. la mise en culture, sur milieu sélectif de l'explant méristématique ayant subi l'étape de transformation ;
c. le prélèvement des bourgeons axillaires transformés et éventuellement des bourgeons foliaires néoformés transformés, obtenus au cours de l'étape b. ;
d. la mise en culture sur milieu sélectif des bourgeons transformés obtenus selon l'étape c. ;
e. la répétition au moins une fois des étapes c. et d. de façon à obtenir des méristèmes secondaires entièrement transformés et éventuellement des méristèmes foliaires néoformés, entièrement transformés ;
f. la régénération, à partir du matériel cellulaire obtenu à l'étape e. de plantes transgéniques.

En d'autres termes, selon l'invention, l'explant ayant subi l'étape de transformation est mis en culture sur milieu sélectif, pendant un temps déterminé. Lorsque des pousses et quelques feuilles apparaissent, la culture est interrompue et les bourgeons axillaires et, le cas échéant les bourgeons foliaires néoformés, qui sont au moins en partie transformés, sont prélevés. L'état transformé se reconnaît grâce au marqueur sélectif. Les bourgeons sont ensuite repiqués et cultivés sur milieu sélectif jusqu'à ce qu'ils produisent, à leur tour, des bourgeons axillaires et éventuellement des bourgeons foliaires néoformés. La culture est interrompue et les bourgeons transformés sont à nouveau prélevés et mis en culture. En répétant plusieurs fois ce cycle de "culture de durée limitée / prélèvement des bourgeons", on parvient à obtenir des bourgeons entièrement transformés. En fait, avec chaque cycle, le nombre de cellules transformées dans le bourgeon et la proportion de cellules transformées par rapport aux cellules non transformées augmente grâce à la multiplication cellulaire. Les cycles sont donc répétés jusqu'à ce que des bourgeons entièrement transformés soient obtenus. Un bourgeon entièrement transformé se reconnaît par sa capacité de produire une pousse qui présente, partout, une coloration verte caractéristique de sa capacité à résister à l'inhibition par le marqueur sélectif, correspondant à l'état transformé. La régénération de la plante transgénique est effectuée à partir des bourgeons axillaires ou des bourgeons foliaires néoformés.

Le terme "agent sélectif" signifie, dans le contexte de l'invention, un agent favorisant le développement des cellules transformées. L'agent sélectif employé est normalement la kanamycine. Dans ce cas, la séquence hétérologue introduite lors de la transformation comprend un gène codant pour la résistance à la kanamycine, par exemple le NPT II. La kanamycine agit par blocage des ribosomes chloroplastiques. Par conséquent, une plante résistante à la kanamycine est verte car elle est capable de fabriquer des chloroplastes fonctionnels, tandis qu'une plante non résistante montre une coloration jaune ou blanche caractéristique de l'absence de chloroplastes fonctionnels. Ce phénomène permet la sélection visuelle des transformants. Les bourgeons foliaires néoformés verts et les bourgeons axillaires verts sont sélectionnés; le bourgeon principal, la plantule principale et toutes les parties de la plante qui sont blanches ou jaunes sont éliminés.

Le fait d'éliminer le bourgeon principal favorise le développement des bourgeons axillaires, particulièrement lorsqu'il s'agit d'une espèce à dominance apicale telle que le tournesol sélectionné.

Le nombre de cycles de culture sélective est de préférence d'au moins 2, y compris la première culture de l'explant méristématique ayant subi l'étape de transformation. Le nombre de cycles doit être suffisant pour permettre l'obtention de bourgeons entièrement transformés. Typiquement, il faut appliquer 3 cycles, mais il pourrait être nécessaire, chez certaines espèces ou avec certaines techniques de transformation, d'en effectuer plus, par exemple 4, 5, ou 6 cycles.

De manière générale, chaque étape de culture sur milieu sélectif dure environ 15 jours, mais peut varier entre 1 et 3 semaines, selon l'espèce. La durée de chaque étape de culture doit être suffisante pour permettre l'apparition de pousses ayant au moins une feuille. Si le cycle de sélection est répété 3 fois, la durée totale de l'étape de culture sélective sera d'environ 6 semaines, l'agent sélectif étant présent pendant la totalité de cette période. Le cas échéant, un gène rapporteur, par exemple GUS, peut également être incorporé lors de la transformation pour permettre l'analyse de l'état de la transformation.

L'efficacité du procédé de la présente invention est inattendue puisque l'ensemble des étapes de culture sélective selon l'invention, a une durée sensiblement plus élevée que celle habituellement pratiquée (par exemple 6 semaines au lieu de 2 semaines). Or, il a été indiqué par plusieurs auteurs que la kanamycine avait des effets néfastes sur la régénération de la plante (Schrammeijer et al, Plant Cell Rep. 9 : 55-60, 1990). Les méthodes de transformation et de régénération, développées jusqu'à ce jour, avaient donc tendance à minimiser la durée de l'étape de sélection (Bidney et al, Proc. 13e Int. Sunflower Conf. Vol II, Pisa Sept. 1992). En fait les présents inventeurs ont démontré qu'une période prolongée de culture sélective sur kanamycine n'exerce aucun effet inhibiteur sur la régénération de la plante, contrairement à ce qui est indiqué dans la littérature.

En ce qui concerne l'étape de transformation de l'explant méristématique, toute technique appropriée peut être employée. Cette étape comprend la mise en contact de l'explant méristématique avec Agrobacterium contenant une séquence hétérologue destinée à être introduite dans les cellules végétales, dans des conditions permettant le transfert de l'ADN.

On peut citer comme exemple de technique de transformation, le bombardement de l'explant méristématique avec des micro-particules, la mise en contact de l'explant avec Agrobacterium étant effectuée soit de manière simultanée, soit après le bombardement. Cette technique permet de réaliser un nombre élevé de micro-blessures sur l'explant, ce qui est nécessaire pour le transfert d'ADN par Agrobacterium. Dans le cas d'un bombardement et d'une transformation simultanée, les micro-particules sont enrobées d'Agrobacterium (EP-A-0486234). De préférence, la mise en contact avec Agrobacterium est effectuée à la suite du bombardement en appliquant par exemple la technique utilisée dans EP-A-0486233. Les micro-particules sont normalement constituées d'or ou de tungstène.

Une autre technique de transformation consiste en la mise en contact de l'explant méristématique avec une suspension d'Agrobacterium. Dans ce cas, il est préférable de réaliser des blessures sur l'explant, par exemple en le coupant avec un scalpel, pour faciliter la transformation.

L'étape de transformation comprend également une période de coculture de l'explant avec l'Agrobacterium. Celle-ci a une d'une durée de 2 à 4 jours, 3 jours étant préférés. Le milieu de co-culture peut être tout milieu normalement utilisé à cette fin. Un milieu particulièrement avantageux est le milieu M2 additionné de BAP, tel que décrit dans les exemples ci-dessous.

L'Agrobacterium est normalement l' Agrobacterium tumefaciens. Diverses souches peuvent être employées, par exemple la souche GV2260 ou LBA 4404. Comme vecteur, on peut citer le plasmide binaire pGA 492-GI, ou encore le p35GUS intron. Le couple "souche/vecteur" peut influencer l'efficacité de la transformation. Il est préférable d'utiliser la souche LBA 4404 avec le vecteur pGA 492-GI.

La séquence d'acide nucléique destinée à être introduite dans les cellules végétales comporte toutes séquences susceptibles de conférer à la plante des traits d'intérêt agronomique. On peut citer comme exemple une séquence conférant une résistance aux insectes, aux herbicides ou aux maladies fongiques (par exemple Sclerotinia sclerotorium ou Botrytis cinerea, un ou plusieurs gènes de protéines de réserve de graines, ou améliorant la qualité des protéines de réserve, des séquences modifiant la maturation du fruit, des séquences conférant une résistance aux virus, un ou plusieurs ribozymes, une ou plusieurs séquences anti-sense, un ou plusieurs gènes impliqués dans le métabolisme des acides gras ou des acides aminés ou un ou plusieurs gènes impliqués dans la stérilité mâle.

En outre, la séquence hétérologue comprend également une séquence codant pour un agent permettant la sélection des transformants, par exemple un agent conférant une résistance à un antibiotique comme la kanamycine, le G418 ou la néomycine. Le vecteur comprend les séquences régulatrices nécessaires pour l'expression stable de la séquence hétérologue. Comme promoteur, on peut citer le promoteur 35S, le 35S double avec l'enhancer de traduction n ou le promoteur de l'ubiquitine provenant du tournesol. Le terminateur de NOS est particulièrement préféré.

Les étapes de transformation et de culture sélective décrites ci-dessus font partie d'un ensemble d'opérations permettant, à partir de l'explant, de régénérer des plantes transgéniques. Le procédé de l'invention, dans son ensemble, peut être résumé ainsi :
1. Préparation de l'explant méristématique ;
2. Mise en oeuvre de l'étape de transformation de l'explant, telle que décrite ci-dessus ;
3. Mise en oeuvre de la série d'étapes de sélection, comme exposée ci-dessus :
4. Régénération de plantes transgéniques à partir des structures obtenues à la fin des cycles de sélection.

Selon le procédé de l'invention, la préparation de l'explant méristématique consiste en une étape de préculture d'un méristème apical, ou d'un demi-méristème apical, pendant 5 à 30 jours, de préférence entre 5 à 8 jours.

Le milieu de préculture est un milieu de culture cellulaire végétale additionné d'une cytokinine et plus particulièrement de la 6-Benzylaminopurine (désignée ci-après comme BAP). De préférence, le milieu est le milieu MS (Murashige-Skoog) additionné de 0.05 à 2.0 mg/l BAP, par exemple 0.1 mg/l de BAP, sans autre hormone.

Les inventeurs ont démontré que l'étape de préculture et sa durée exercent une influence importante sur le développement de l'explant. Lorsqu'elle a une durée supérieure à 9 ou 10 jours, des bourgeons foliaires néoformés apparaissent sur les feuilles. Les pousses régénérées sur le méristème apical ont alors des feuilles qui portent des bourgeons foliaires néoformés sur l'épiderme supérieur.

Selon cette variante de l'invention, il est possible, avant l'étape de transformation, d'exciser les bourgeons foliaires néoformés ou même simplement de prélever des morceaux de feuille, et de les utiliser dans l'étape de transformation. Selon ce mode de réalisation de l'invention, l'explant méristématique soumis à la transformation peut donc être constitué soit par une partie d'une feuille obtenue après au moins 9 jours de préculture susceptible de donner des bourgeons foliaires néoformés, soit par des bourgeons foliaires néoformés excisés.

Dans le cas où la préculture dure entre 5 et 12 jours, le méristème apical sert normalement directement d'explant méristématique dans l'étape de transformation. Dans ce cas, les bourgeons foliaires néoformés sont induits pendant l'étape de préculture proprement dite, mais n'apparaissent qu'au cours du développement ultérieur, par exemple pendant la co-culture ou pendant la culture sur milieu sélectif.

Selon l'invention, le même milieu de culture peut être utilisé pour les étapes de préculture, de coculture et de sélection. Ce milieu est de préférence le milieu MS additionné de BAP à une concentration de 0.05 à 2.0 mg/l, de préférence 0.1 mg/l. Normalement, la BAP est la seule phyto-hormone présente dans le milieu. En particulier, il est préférable que ce milieu ne contienne ni d'acide gibbéréllique ni d'acide indolacétique, particulièrement lorsqu'il s'agit du milieu de préculture. Le milieu peut également être additionné, pendant les étapes de préculture et de co-culture, d'un composé phénolique, par exemple, l'acétosyringone, capable d'activer les gènes vir d'Agrobacterium. Une concentration d'environ 200 µM est appropriée. Pour l'étape de culture sélective, le milieu contient au moins un agent sélectif, avantageusement la kanamycine, à une concentration comprise entre 50 et 200 mg/l, de préférence 50 mg/l, et éventuellement, des agents bactériostatiques.

Les méristèmes apicaux sont obtenus par la germination de graines mûres décortiquées et stérilisées. Le procédé de germination consiste typiquement en la mise en culture des graines sur un milieu de germination, de préférence solidifié, par exemple le milieu MS dont les macro- et micro-éléments ont été éventuellement réduits par moitié. La germination a une durée comprise entre 2 à 4 jours, de préférence 4 jours, à 25°C de préférence avec une photo-période de 16 heures environ.

Après les étapes de préculture, de transformation et de sélection, une étape de régénération est effectuée à partir des bourgeons et des pousses entièrement transformés. Les milieux de culture et les conditions sont ceux habituellement appliqués dans l'art pour l'espèce en question. Pour le tournesol, les conditions de régénération sont indiquées dans les exemples ci-dessous.

Outre le procédé de production de plantes transgéniques décrit ci-dessus, la présente demande concerne également les bourgeons foliaires néoformés et les méristèmes secondaires entièrement transformés obtenus au cours du procédé. Elle vise également les plantes transgéniques entièrement transformées en génération T₀ obtenues à partir des explants méristématiques.

Le procédé de l'invention présente de nombreux avantages. Il est notamment, plus efficace que les techniques de l'art antérieur puisque la régénération n'est effectuée que si l'explant est entièrement transformé. Les techniques existantes impliquaient, en revanche, la régénération de tous les méristèmes ayant subi l'opération de transformation, suivie de la sélection, parmi les plantes chimériques obtenues, de celles dont la lignée germinale avait été transformée. Le procédé de l'invention permet donc un gain de temps et de moyens important.

De plus, le rendement, en plantes transgéniques, obtenu selon ce procédé est considérablement plus élevé que celui obtenu selon les techniques antérieures. Par exemple, pour le tournesol, 92 % des plantes régénérées donnent au moins une plante transgénique dans la descendance (tableau 8 ci-dessous). Ce chiffre est à comparer à 0.2 % à 2 % rapporté par Bidney et al, Plant Molecular Biol. 18, 301-313, 1992.

Le procédé de l'invention s'applique à de nombreuses espèces végétales et plus particulièrement aux dicotylédons, notamment à celles qui sont difficiles à transformer et à régénérer. On citera comme exemple le coton, le soja, les espèces végétales oléagineuses, par exemple le tournesol, les espèces appartenant à la famille des légumineuses, par exemple le pois, le haricot ou encore les espèces appartenant à la famille des cucurbitacées, par exemple la courgette. Au sein de ces espèces, de nombreux génotypes différents peuvent être employés. Le tournesol est particulièrement préféré.

Différents aspects de l'invention sont illustrés dans les figures :

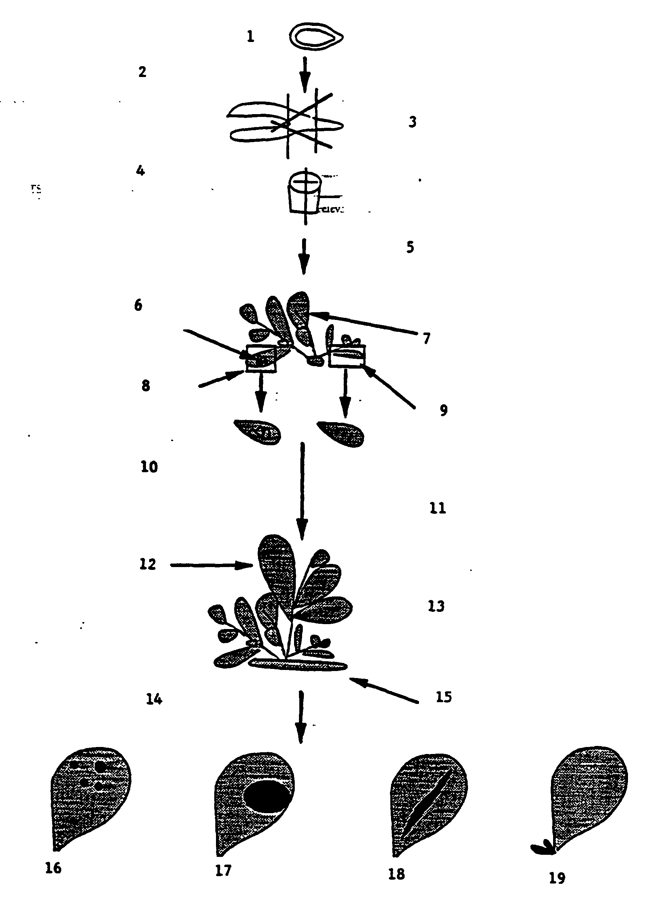

La figure 1 montre le protocole de transformation de feuilles issues de demi-méristèmes pour l'analyse de l'expression de la Glucuronidase dans les pousses axillaires régénérées à partir de bourgeons foliaires néoformés.

### Légende :

1 - graine
2 - germination milieu M0, 2 jours
3 - dissection des apex : élimination des cotylédons, de la radicule et de la première feuille
4 - section de l'apex en deux moitiés
5 - préculture des demi-méristèmes sur le milieu M2 contenant de l'acétosyringone à 200 µM pendant 30 jours
6 - bourgeon foliaire néoformé
7 - pousses axillaires induites à partir de demi-méristèmes
8 - prélèvement des feuilles portant des bourgeons foliaires néoformés initiés sur la face supérieure
9 - prélèvement des feuilles sans bourgeon foliaire néoformé
10 - bombardement et 3 jours de coculture avec Agrobactérium tumefaciens des feuilles sur le milieu M2 contenant de l'acétosyringone à 200 µM
11 - culture sur le milieu M2 contenant de l'augmentin à 400 mg/l pendant 15 jours
12 - pousses axillaires
13 - développement de bourgeons foliaires néoformés en pousses axillaires
14 - analyses de l'expression de la glucuronidase dans les pousses axillaires induites à partir de bourgeons foliaires néoformés apparus sur les feuilles
15 - explant foliaire
16 - spots de cellules transformées GUS+
17 - secteurs cellulaires transformés GUS+
18 - lignées cellulaires transformées GUS+
19 - bourgeons axillaires transformés GUS+

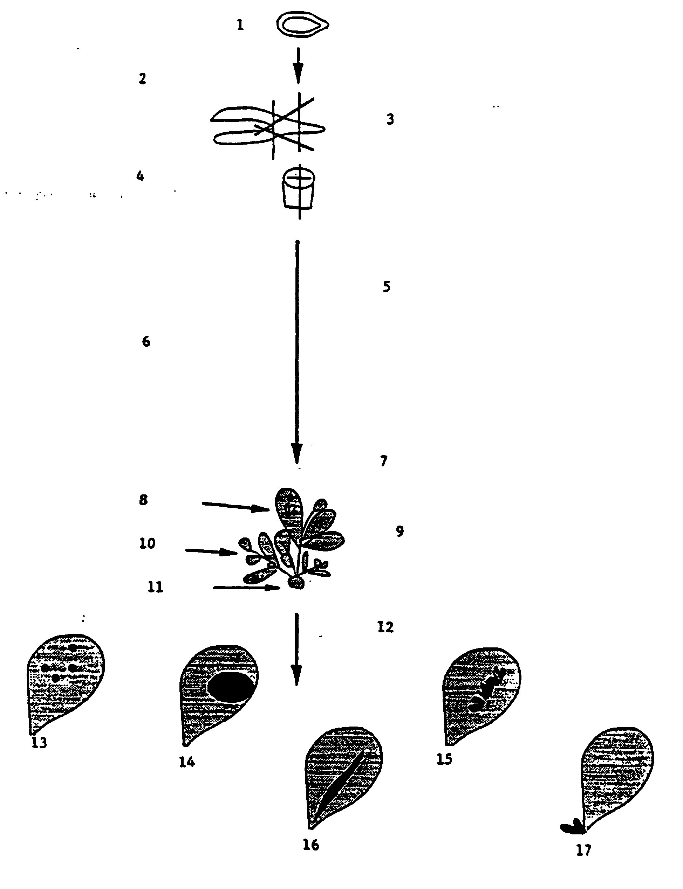

La figure 2 montre le protocole de transformation de demi-méristèmes pour l'analyse de l'expression de la Glucuronidase.

### Légende :

1 - graine
2 - germination milieu MO, 2 jours
3 - dissection des apex : élimination des cotylédons, de la radicule et de la première feuille
4 - section de l'apex en deux moitiés
5 - préculture des demi-méristèmes sur le milieu M2 contenant de l'acétosyringone à 200 µM pendant 5 jours
6 - bombardement et 3 jours de coculture avec Agrobactérium tumefaciens des demi-méristèmes sur le milieu M2 contenant de l'acétosyringone à 200 µM
7 - culture des demi-méristèmes bombardés et cocultivés sur le milieu M2 contenant de l'augmentin à 400 mg/l pendant 15 jours
8 - bourgeon foliaire néoformé
9 - pousses axillaires induites à partir des demi-méristèmes
10 - pousse axillaire
11 - cal à la base
12 - analyses de l'expression de la glucuronidase dans les pousses axillaires induites à partir de demi-méristèmes
13 - spots de cellules transformées GUS+
14 - secteurs cellulaires transformés GUS+
15 - bourgeons foliaires néoformés transformés GUS+
16 - lignées cellulaires transformées GUS+
17 - bourgeons axillaires transformés GUS+

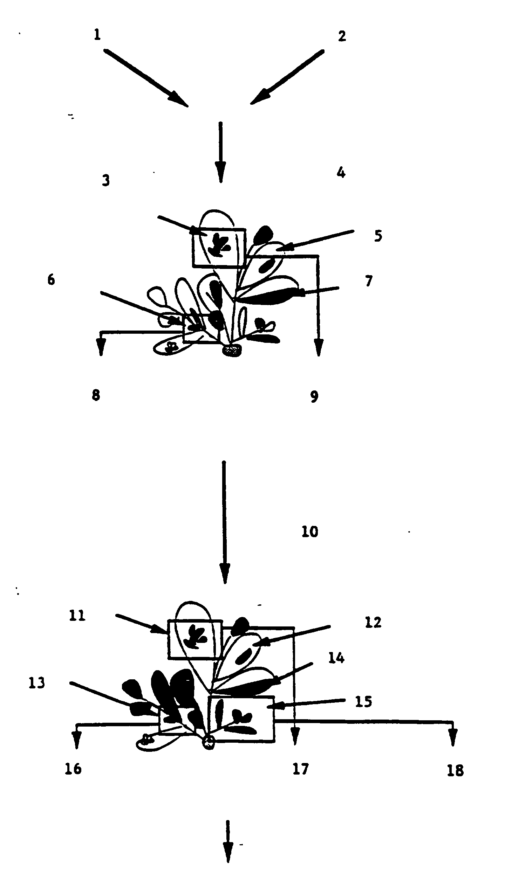

La figure 3 montre la sélection de pousses axillaires transformées et régénérées à partir de bourgeons foliaires néoformés sur les feuilles ou à partir de demi-méristèmes.

### Légende :

1 - demi-méristèmes bombardés et cocultivés selon le protocole défini dans l'exemple 2
2 - feuilles issues de demi-méristèmes, bombardées et cocultivées selon le protocole défini dans l'exemple 1
   Première culture en sélection sur kanamycine :
3 - bourgeon foliaire néoformé vert résistant à kanamycine
4 - culture des explants sur le milieu M2 contenant de l'augmentin à 400 mg/l et de la kanamycine à 50 mg/l pendant 15 jours
5 - partie blanche non transformée sensible à la kanamycine
6 - bourgeon axillaire vert résistant à la kanamycine
7 - partie verte transformée résistante à la kanamycine
8 - prélèvement des bourgeons axillaires verts
9 - prélèvement des bourgeons foliaires néoformés verts Deuxième culture en sélection sur kanamycine :
10 - culture des bourgeons axillaires verts, des bourgeons foliaires néoformés verts et des pousses axillaires vertes sur le milieu M2 contenant de l'augmentin à 400 mg/l et de la kanamycine à 50 mg/l pendant 15 jours
11 - bourgeon foliaire néoformé vert résistant à la kanamycine
12 - partie blanche non transformée sensible à la kanamycine
13 - bourgeon axillaire vert résistant à la kanamycine
14 - partie verte résistante à la kanamycine
15 - pousse axillaire verte résistante à la kanamycine
16 - prélèvement des bourgeons axillaires verts
17 - prélèvement des bourgeons foliaires néoformés verts
18 - prélèvement des pousses axillaires vertes

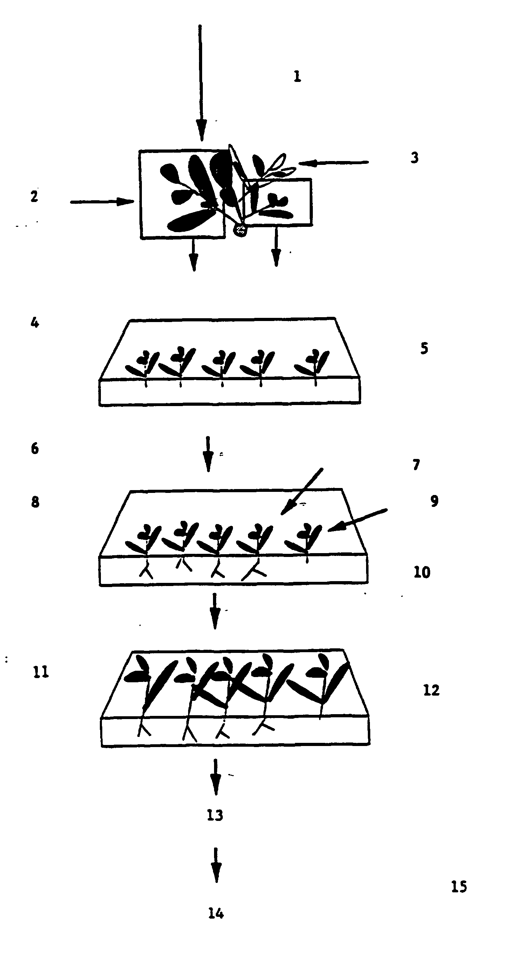

La figure 4 montre la régénération de plantes transformées.

### Légende :

Troisième culture en sélection sur kanamycine :
   1 - culture des bourgeons axillaires verts, des bourgeons foliaires néoformés verts et des pousses axillaires vertes sur le milieu M2 contenant de l'augmentin à 400 mg/l et de la kanamycine à 50 mg/l pendant 15 jours
   2 - prélèvement des pousses axillaires vertes
   3 - pousse axillaire chimérique avec des parties blanches non transformées et des parties vertes transformées
   4 - culture dans le système SORBAROD additionné de milieu M3 pendant 30 jours en chambre de culture
   5 - enracinement
   6 - lavage du milieu M3, addition de milieu M4
   7 - plante enracinée
   8 - culture dans le système SORBAROD pendant 10 jours en chambre de culture
   9 - plante non enracinée
   10 - développement
   11 - culture dans le système SORBAROD en serre pendant 10 jours
   12 - développement et acclimatation
   13 - repiquage en tourbe dans des pots des plantes enracinées , greffage sur porte-greffes des plantes non-enracinées, culture en serre
   14 - floraison, autofécondation
   15 - croissance

### Exemple 1 : Transformation de feuilles issues de demi-méristèmes pour l'analyse de l'expression de la glucuronidase dans les pousses régénérées à partir de bourgeons foliaires néoformés (Figure 1).

### 1.1 - Préparation des demi-méristèmes

Les graines de tournesol (Helianthus annuus) des lignées (Ha300, RHa 274, RHa 297, RHa 356, RHa 359, RHa 362), deux populations LG60 et LG61 dérivées de croisements interspécifiques avec Helianthus petiolaris, et plusieurs lignées fournies par LIMAGRAIN GENETICS (Les Alleuds) sont utilisées pour ces expériences. Les graines sont décortiquées pour éliminer les téguments, puis stérilisées pendant 20 minutes dans de l'eau de javel à 12° de chlore additionnée de 1 ml/l de Tween 80. Les graines décortiquées sont ensuite rincées 5 fois à l'eau distillée stérile. Les graines sont ensuite mises à tremper 1 heure dans de l'eau distillée stérile, puis la pellicule recouvrant la graine est éliminée. Les graines nues sont à nouveau stérilisées 2 minutes dans de l'eau de javel à 6° chlore, rincées 3 fois à l'eau distillée stérile et séchées sur papier filtre dans la hotte à flux laminaire. Les graines sèches sont ensuite mises à germer sur le milieu M0 : macro-éléments, micro-éléments, Fe-EDTA et vitamines du milieu MS (Murashige et Skoog, 1962 - Physiologia Plantarum, 15 : 473-497) additionné de 10 g/l de saccharose, de 7 g/l d'agar-agar, pH 5,8 ; et mises à germer dans une chambre de culture à 26° avec 16 h de lumière (50 µ Einstein / m2 d'intensité lumineuse), pendant deux jours. Les graines germées sont prélevées, les cotylédons et les radicules sont éliminés ainsi que la partie apicale de l'apex. Le cylindre de quelques millimètres de diamètre ainsi obtenu est ensuite coupé en deux moitiés dans l'axe des insertions cotylédonaires. Cet explant contenant un demi-méristème apical est appelé demi-méristème.

### 1.2 - Culture des demi-méristèmes

Les demi-méristèmes sont ensuite mis en culture dans le milieu M2 (macro-éléments, micro-éléments, Fe-EDTA et vitamines du milieu MS) additionné de 10 g/l de saccharose, de 0,1 mg/l de BAP, 8 g/l d'agar-agar, pH : 5,8 à 6) contenant 200 µM d'acétosyringone pendant 30 jours dans une salle de culture à 26°C avec 16 h de lumière (50 µ Einstein/m2 d'intensité lumineuse). Pendant cette période de culture les demi-méristèmes développent des pousses provenant des méristèmes secondaires à l'aisselle des primordia foliaires. Après 10 jours de culture, des protubérances apparaissent à la face supérieure des feuilles. Ces protubérances, qui sont des méristèmes foliaires néoformés se transforment en bourgeons foliaires néoformés (Figure 1). Ces bourgeons sont issus de néoformations de méristèmes végétatifs à partir des cellules épidermiques et sous épidermiques de la face supérieure des feuilles.

Après 30 jours de culture, les feuilles présentant des méristèmes secondaires ou des bourgeons foliaires néoformés et les feuilles sans bourgeons foliaires néoformés sont prélevées (Figure 1).

### 1.3 - Bombardement et coculture des feuilles issues de demi-méristèmes.

Ces feuilles sont bombardées avec un canon à particules à hélium (Particle Inflow Gun, PIG) (FINER et al, 1992 - Plant Cell Reports, 11 : 323-328) avec des particules de tungstène (0,2 µm à 2 µm de diamètre) pour réaliser des micro-blessures sur les explants.

Les particules de tungstène (50 mg) sont stérilisées dans 500 µl d'éthanol à 95 % pendant 20 min puis rincées 4 fois par centrifugation dans de l'eau stérile (10 000 tr/min pendant 1 min). Les particules stériles sont reprises dans 300 µL de tampon TE (Tris HCl 10 mM, EDTA 1 mM). Un volume de 2 µl de particules nues est utilisé pour chaque bombardement. Les feuilles sont déposées face supérieure vers le haut sur une boite de Pétri de 5 cm de diamètre contenant de l'eau gélosée à 15 g/l. Les boites de Pétri contenant les explants sont placées dans l'enceinte du canon et un vide de 29 inchs de Hg est réalisé. Le bombardement des particules est fait avec une pression de 8 bars à une distance de 16 cm entre le canon et les explants à bombarder.

Les explants bombardés sont ensuite placés dans une boite de Pétri de 9 cm de diamètre contenant du milieu M2 additionné de 200 µM d'acétosyringone. Une goutte (1 à 10 µl) d'une suspension d'Agrobacterium tumefaciens LBA 4404 désarmé (HOEKEMA et al, 1983 - Nature, 303 : 179-180) provenant d'une culture over-night à une densité optique Do = 2 à 600 nm est déposée sur l'explant. La souche LBA 4404 contient le vecteur binaire pGA492-GI (AN, 1986 - Plant Physiol., 81 : 86-91) porteur du gène NPT II conférant la résistance à la kanamycine sous contrôle du promoteur pNOS (HERRERA-ESTRELLA et al, 1983 - EMBO J., 2 : 987-995) et du terminateur t-NOS (DEPICKER et al, 1982 - Mol. Appl. Genet., 1 : 561-573) dans lequel a été inséré au site Sca I, le gène GUS intron sous le contrôle du promoteur p35S du CaMV et du terminateur t-NOS (VANCANNEYT et al, 1990 - Mol. Gen. Genet., 220 : 245-250). Les explants bombardés et inoculés avec Agrobacterium tumefaciens sont cocultivés pendant 3 jours à 26°C avec 16 h de lumière (50 µ Einstein /m²). L'association du bombardement avec des particules nues et la coculture avec Agrobacterium tumefaciens permet de provoquer par le bombardement des micro-blessures dans les cellules des feuilles et dans les méristèmes secondaires. Ces micro-blessures assurent la pénétration de la bactérie et une plus grande efficacité de transformation selon le procédé de BIDNEY et al, 1992 (Plant Mol Biol., 18 : 301-313).

### 1.4 - culture des feuilles et régénération de pousses a partir de bourgeons foliaires néoformés

Après la coculture, les explants sont repiqués dans une boite de Pétri contenant du milieu M2 additionné d'augmentin à 400 mg/l et placés pendant 15 jours en chambre de culture (16 h de lumière, 15 µ Einstein/m2, 23°C et 8 h d'obscurité, 21°C).

Après 15 jours de culture, les bourgeons foliaires néoformés apparus ou déjà présents sur les feuilles bombardées et cocultivées, se sont développés en pousses possédant 1 à 5 feuilles et une tige courte. Ces pousses sont séparées et placées dans un tampon pour l'analyse de l'activité glucuronidase (Figure 1).

### 1.5 - Analyse de l'activité glucuronidase dans les pousses issues de bourgeons foliaires néoformés.

Les pousses issues de bourgeons foliaires néoformés sont immergées dans un tampon GUS contenant 1 mg/ml de X-gluc. (5 bromo-4 chloro - 3 indolyl-β-D-glucuronic acid cyclohexylammonium) (JEFFERSON, 1987 - Plant Mol. Biol. Rep., 5 : 387-405) infiltrées sous vide et incubées 24 h à 37°C à l'obscurité.

Après 24 h d'incubation, les cellules transformées qui ont exprimé le gène GUS, possèdent une activité glucuronidase (notées activité GUS +), sont capables d'hydrolyser le X-Glu et libèrent un composé de couleur bleue.

Différents types de plages de cellules transformées ayant une activité GUS + sont visibles après une incubation de 24 h (Figure 1).
- Des spots de quelques millimètres de diamètre localisés à la surface des feuilles des pousses régénérées à partir de méristèmes foliaires néoformés. Ces spots proviennent de cellules de méristèmes foliaires néoformés transformées au moment du bombardement et de la coculture.
- Des larges secteurs de cellules transformées pouvant être soit des disques bleus de diamètre supérieur à 5 mm soit des demi-feuilles bleues, soit des lignées de cellules bleues. Ces larges secteurs proviennent de cellules transformées situées à l'intérieur des méristèmes des bourgeons foliaires néoformés qui ont donné des descendances cellulaires entièrement transformées (demi-feuilles - disques et lignées cellulaires).
- Dans quelques cas, des bourgeons ou méristèmes secondaires bleus exprimant la glucuronidase sont observés à l'aisselle des feuilles des pousses. Ces méristèmes secondaires sont entièrement transformés. Ils proviennent soit de cellules internes des méristèmes foliaires néoformés bombardés et cocultivés qui ont donné au cours de la croissance et de la différenciation un bourgeon axillaire à l'aisselle d'une feuille, soit de cellules transformées à la surface de la feuille qui a néoformé des méristèmes foliaires néoformés puis un bourgeon secondaire.

Les résultats du tableau 1 illustrent le pourcentage de feuilles bombardées et cocultivées ayant donné des pousses présentant soit des spots de cellules GUS +, soit des larges secteurs de cellules GUS +, soit des méristèmes secondaires GUS. La plupart des explants foliaires bombardés régénèrent des pousses à partir de bourgeons foliaires néoformés qui contiennent des spots GUS + (80 %), et des larges secteurs GUS + (55 %). Ces pousses sont des plantules chimériques composées d'un mélange de cellules transformées et non transformées.

| Pourcentage d'explants bombardés et cocultivés présentant des : | | |
|---|---|---|
| spots GUS+ | larges secteurs GUS+ | bourgeons axillaires |
| 80 | 55 | 2 |

Ces pousses donneront une descendance transformée génétiquement seulement s'il existe des cellules transformées dans la zone du méristème contenant les cellules germinales à l'origine des ovules ou des grains de pollen. Par contre, 2 % des explants foliaires bombardés et cocultivés contiennent des bourgeons axillaires transformés situés à l'aisselle des feuilles portées par les pousses issues de bourgeons foliaires néoformés. L'origine unicellulaire de ces bourgeons axillaires implique qu'ils sont entièrement transformés et donneront une descendance de plantes transformées suivant la ségrégation Mendélienne (au moins 3/4 de plantes transformées et 1/4 de plantes non transformées).

### Exemple 2 : Transformation de demi-méristèmes pour l'analyse de l'expression de la glucuronidase dans les pousses régénérées à partir de demi-méristèmes (Figure 2).

### 2.1. Préparation des demi-méristèmes

La préparation des demi-méristèmes est identique à celle présentée dans l'exemple 1 (partie 1.1)

### 2.2. Préculture des demi-méristèmes

Les demi-méristèmes sont mis en préculture pendant 1, 5, 8 et 12 jours dans le milieu M2 contenant de l'acétosyringone à 200 µM à 26°C avec 16 h de lumière (50 µ Einstein / m²).

Après la préculture les demi-méristèmes forment de petites pousses développées à partir des méristèmes secondaires et du méristème apical. Le développement de ces pousses est fonction de la durée de préculture. Plus la période de préculture est longue, plus les pousses sont développées. A 12 jours de préculture, les pousses portent des feuilles et des bourgeons foliaires néoformés à la surface supérieure des feuilles (Figure 2).

### 2.3 - Bombardement et coculture des demi-méristèmes précultivés

Après la préculture, les demi-méristèmes sont bombardés et cocultivés selon le protocole décrit dans l'exemple 1 partie 1.3.

### 2.4 - Culture des demi-méristèmes bombardés et cocultivés.

Les demi-méristèmes une fois précultivés, bombardés et cocultivés sont mis en culture dans des boites de Pétri de 9 cm de diamètre contenant du milieu M2 additionné de 400 mg/l d'augmentin et placés dans une chambre de culture (16 h de lumière, 15 µ Einstein / m², 23°C et 8 h d'obscurité, 21°C).

Après 15 jours de culture, plusieurs pousses possédant 1 à 5 feuilles et une tige se développent à partir des bourgeons axillaires, du méristème apical et aussi des bourgeons foliaires néoformés apparus à la surface supérieure des feuilles.

### 2.5 - Analyses de l'activité glucuronidase dans les pousses issues de demi-méristèmes (Figure 2)

Les pousses formées à partir des demi-méristèmes sont séparées et analysées pour l'expression de l'activité glucuronidase. Les conditions du test de l'activité glucuronidase sont décrites dans l'exemple 1 partie 1.5. Des plages de cellules ayant une activité glucuronidase sont observés (Figure 2) :
- des spots de cellules transformées bleus exprimant la glucuronidase (GUS +)
- des larges secteurs ou des lignées cellulaires exprimant la glucuronidase (GUS +)
- des méristèmes secondaires à l'aisselle des feuilles entièrement bleus exprimant la glucuronidase (GUS +)
- de plus, dans le cas des transformations de demi-méristèmes précultivés (1 à 12 jours) des bourgeons foliaires néoformés transformés GUS + apparaissent à la face supérieure des feuilles (Figure 2). Ces bourgeons foliaires néoformés sont issus de cellules transformées localisées sur les jeunes feuilles au moment du bombardement et de la coculture du demi-méristème. Ces cellules se sont ensuite développées en bourgeons foliaires néoformés. L'origine unicellulaire de ces bourgeons foliaires néoformés permet d'obtenir des bourgeons entièrement transformés qui donneront des plantes entièrement transformées.

| | | | |
|---|---|---|---|
| Durée de préculture | Pourcentage de demi-méristèmes bombardés et cocultivés présentant des : | | |
| | spots de cellules GUS+ | larges secteurs GUS+ | bourgeons axillaires GUS+ |
| 1 jour | 38 | 26 | 0 |
| 5 jours | 50 | 40 | 4 |
| 8 jours | 67 | 47 | 0 |
| 12 jours | 38 | 38 | 0 |

Les fréquences de transformation obtenues par bombardement de demi-méristèmes sont élevées, 38 à 70 % de demi-méristèmes bombardés et cocultivés présentent des spots GUS + et 26 à 47 % présentant des larges secteurs GUS + (Tableau 2).

Les pourcentages de demi-méristèmes bombardés et cocultivés présentant de larges secteurs GUS + sont les plus élevés lorsque les demi-méristèmes sont précultivés 5 jours (40 %), et 8 jours (47 %) (Tableau 2).

Seuls les demi-méristèmes précultivés 5 jours ont donné des pousses avec des bourgeons axillaires ou foliaires néoformés entièrement bleus.

Ces bourgeons axillaires ou foliaires néoformés entièrement transformés donneront des plantes entièrement transformées dont la descendance sera au moins de 3/4 de plantes transformées et 1/4 de plantes non transformées. Une durée de 5 jours de préculture est donc la durée optimale pour le protocole standard décrit dans cette invention.

### Exemple 3 : Transformation de demi-méristèmes ou de feuilles issues de demi-méristèmes (Figures 3 et 4).

### 3.1 - Transformation de feuilles issues de demi-méristèmes.

La préparation et la préculture des demi-méristèmes, le bombardement et la coculture des feuilles issues des demi-méristèmes se font selon le protocole décrit dans l'exemple 1 (parties 1.1, 1.2 et 1.3)

### 3.2 - Transformation de demi-méristèmes

La préparation, la préculture, le bombardement et la coculture des demi-méristèmes se font selon le protocole décrit dans l'exemple 2 (parties 2.1, 2.2, 2.3).

### 3.3 - Sélection des pousses résistantes à la kanamycine

### 3.3.1 - Première culture en sélection en présence de kanamycine (Figure 3)

Après la coculture, les explants (demi-méristèmes ou feuilles) sont placés dans une boite de Pétri de 9 cm de diamètre contenant le milieu M2 additionné de 400 mg/l d'augmentin et de 50, 100, 200 et 400 mg/l de kanamycine.

Les boites contenant les explants sont placées dans une chambre de culture (16 h de lumière, 15 µ Einstein / m2, 23°C et 8 h d'obscurité, 21°C) pendant 15 jours de culture. Au cours de cette culture, les explants se développent pour donner des pousses possédant 1 à 5 feuilles et une tige courte (Figure 3).

Après 15 jours de culture sur le milieu sélectif contenant de la kanamycine, les pousses régénérées présentent des aspects différents selon la quantité de cellules transformées résistantes à la kanamycine qui les composent :
- des pousses portant des feuilles avec des secteurs blancs ou jaunes composées de cellules non transformées sensibles à la kanamycine et de secteurs verts composés de cellules transformées résistantes à la kanamycine.
- des pousses portant des bourgeons axillaires verts à l'aisselle des feuilles en cours de développement. Ces bourgeons axillaires verts sont transformés et se développent en pousses axillaires en présence de kanamycine.
- des pousses portant sur leurs feuilles des bourgeons foliaires néoformés jaunes ou blancs non transformés, sensibles à la kanamycine.
- des pousses entièrement blanches ou jaunes non transformées, sensibles à la kanamycine
- des pousses portant sur les feuilles des bourgeons ou des méristèmes foliaires néoformés verts en développement, résistants à la kanamycine.

### 3.3.2 - Deuxième culture en sélection en présence de kanamycine (Figure 3)

Les pousses axillaires vertes, les bourgeons axillaires verts et les méristèmes ou bourgeons foliaires néoformés verts sont repiqués sur le milieu M2 contenant de l'augmentin à 400 mg/l et de la kanamycine (50-100-200 et 400 mg/l) et incubés dans les mêmes conditions de culture que précédemment (partie 3.3.1) pendant 15 jours.

Après cette période de culture, les bourgeons axillaires et les bourgeons foliaires néoformés ont de nouveau développé des pousses (Figure 3)
- certaines pousses non transformées sont entièrement blanches indiquant une sensibilité à la kanamycine.
- certaines pousses ont des feuilles portant des parties vertes composées de cellules transformées résistantes à la kanamycine et des parties blanches composées de cellules non transformées sensibles à la kanamycine.

La présence de pousses, non entièrement transformées indique qu'à l'issue de la première culture tous les bourgeons axillaires et bourgeons foliaires néoformés verts n'étaient pas entièrement transformés et ont donné naissance à des pousses chimériques composées d'un mélange de cellules transformées et non transformées.
- certaines pousses chimériques ont des méristèmes secondaires ou bourgeons axillaires verts résistants à la kanamycine.
- certaines pousses chimériques possèdent des feuilles avec les plages vertes et des plages blanches qui portent des bourgeons ou méristèmes foliaires néoformés verts résistant à la kanamycine.
- certaines pousses ont des feuilles vertes portant des bourgeons axillaires ou des méristèmes secondaires verts. Ces pousses composées de cellules transformées sont résistantes à la kanamycine.

Ces pousses entièrement vertes provenant des bourgeons axillaires ou des bourgeons ou méristèmes foliaires néoformés se développent. Ces pousses sont composées d'un grand nombre de cellules transformées résistantes à la kanamycine.

Dans les pousses obtenues à l'issue de la seconde culture, un certain nombre de pousses entièrement vertes sont observées alors qu'à l'issue de la première culture, aucune pousse entièrement verte n'était observée, ce qui indique que certains bourgeons axillaires ou foliaires néoformés cultivés dans la deuxième culture en présence de kanamycine étaient entièrement transformées.

### 3.3.3 - Troisième culture en sélection en présence de kanamycine (Figure 4)

Les pousses entièrement vertes, les bourgeons axillaires verts, et les bourgeons ou méristèmes foliaires néoformés verts sont séparés et repiqués dans le milieu M2 et cultivés comme précédemment (partie 3.3.1) (Figures 3 et 4).

Après 15 jours de culture des pousses se sont développées à partir de ces explants. La plupart des pousses sont entièrement vertes et se développent rapidement sur le milieu M2 contenant de la kanamycine. Ces pousses sont entièrement transformées. Un petit nombre de pousses présentent encore des plages vertes et des plages de cellules blanches. Ces pousses chimériques sont éliminées (Figure 4).

Seules les pousses vertes sont repiquées dans un système de culture SORBAROD. Le système de culture SORBAROD consiste en une mini-serre fermée et stérile contenant des cylindres de cellulose humidifiés avec du milieu M3 (macro-éléments, micro-éléments, Fe-EDTA et vitamines du milieu MS) additionné de 10 g/l de saccharose. La mini-serre est perforée de 3 orifices recouverts d'une membrane assurant les échanges gazeux et équilibrant l'humidité relative intérieure avec l'humidité relative extérieure.

Les pousses vertes issues de la troisième culture sont plantées dans des cylindres de cellulose et installées dans les mini-serres SORBAROD (Figure 4). Les mini-serres sont placées dans une chambre de culture (16 h lumière 23°C, 15 µ Einstein / m2, et 8 h d'obscurité, 21°C). Après 30 jours d'incubation, les plantes transformées s'enracinent et se développent. Le milieu M3 est ensuite éliminé par pipettage et les cylindres sont rincés 3 fois à l'eau distillée stérile puis les cylindres sont humectés avec du milieu M4 (macro-éléments, micro-éléments, Fe-EDTA et vitamines du milieu MS) dépourvu de saccharose. Après 10 jours de culture en chambre de culture, les plantes développent de larges feuilles et atteignent une taille de 2 à 3 cm. Les mini-serres SORBAROD sont transférées en serre (température 26°C - photopériode 16 h / 8 h) pendant 10 jours. Pendant cette période, les plantes enracinées se développent et s'acclimatent au conditions de serre. Ensuite, les cylindres portant les plantes de tournesol ayant développé des racines et des feuilles sont placées dans des pots contenant de la tourbe arrosée avec une solution de type COIC et LESAINT (1971 - Hortic. Fr., 8 : 11).

Les plantes non enracinées sont greffées sur des tiges de porte-greffes (tournesol non transformé stade 4 à 6 feuilles après la levée). La base de la tige de la plante transformée est coupée en biseaux et placée dans une fente réalisée sur la tige du porte-greffe. Le greffon est fixé au porte-greffe par un lien de raffia et entouré d'un sac plastique. Après la prise du greffon, les feuilles et la tige se développent et le sac plastique est enlevé. Les porte-greffes et les greffons sont ensuite conduits en serre avec un arrosage avec la solution nutritive de type COIC et LESAINT (1971 - Hortic. Fr., 8 : 11).

Les plantes de tournesol croissent, fleurissent et sont autofécondées. Les graines de chaque plante transformée sont récoltées pour analyse de la ségrégation du gène de résistance à la kanamycine et du gène GUS (voir, exemple 8).

### Exemple 4 : Influence de la souche bactérienne et du plasmide binaire sur l'efficacité de transformation génétique des feuilles issues de demi-méristèmes.

Les feuilles issues de demi-méristèmes sont bombardées et cocultivées avec différentes souches :
- LBA 4404 contenant le plasmide binaire PGA 492-GI (décrit dans l'exemple 1, partie 1.3)
- GV 2260 contenant le plasmide binaire p35S GUS intron (VANCANNEYT et al, 1990 - Mol. Gen. Genet, 220 : : 245-250)
- C58'3 (MULLINEAU et al, 1989 - Plant Sci, 63 : 237-245) contenant le plasmide pGA 492-GI (Cf exemple 1, partie 1.3). ,

Après 6 semaines de culture des feuilles (selon le protocole décrit dans l'exemple 3) sur milieu sélectif contenant 100 mg/l ou 200 mg/l de kanamycine, les pousses vertes résistantes à la kanamycine sont soumises à un test d'expression de l'activité glucuronidase. Lorsque les cellules expriment l'activité glucuronidase, cette activité est notée activité GUS +. Le protocole pour tester l'activité glucuronidase est décrit dans l'exemple 1, partie 1.5.

Les résultats sont présentés dans le tableau 3.

| Souches et plasmides binaires | % d'explants présentant des : | | |
|---|---|---|---|
| | spots GUS+ | larges secteurs GUS+ | bourgeons axillaires GUS+ ou des pousses entièrement GUS+ |
| GV 2260 (pGUS intron) | 21.5 | 4.5 | 0 |
| C58'3 (pGA 492-GI) | 0 | 0 | 0 |
| LBA 4404 (pGA 492-GI) | 38.5 | 18.5 | 1 |

Seules les pousses régénérées provenant des cocultures avec GV 2260 (pGUS intron) et LBA 4404 (pGA 492 GI) montrent une expression de la glucuronidase (activité GUS+). Avec la souche C58'3 aucune activité GUS + n'a été détectée. Le pourcentage d'explants bombardés et cocultivés montrant au moins une pousse avec de larges secteurs exprimant une activité GUS + est 4 fois plus élevé lorsque les explants sont cocultivés avec la souche LBA 4404 (pGA 492-GI) que lorsqu'ils sont cocultivés avec la souche GV 2260 (pGUS-intron). Seule la souche LBA 4404 permet d'obtenir des pousses ou des méristèmes secondaires présentant une activité GUS + dans toutes les cellules.

### Exemple 5 : Influence de la souche bactérienne et du plasmide binaire sur l'efficacité de transformation génétique de demi-méristèmes.

Les demi-méristèmes sont bombardés et cocultivés avec les souches LBA 4404 (pGA 492-GI), C58'3 (pGA 492-GI) et GV 2260 (p35S GUS Intron) décrites dans l'exemple 4.

Après 6 semaines de culture selon le protocole décrit dans l'exemple 3, le nombre de demi-méristèmes capables de développer des pousses en présence de kanamycine (100 mg/l) a été comptabilisé (Tableau 4).

Les résultats sont les suivants :

L'efficacité de transformation est définie comme étant le pourcentage de demi-méristèmes bombardés et cocultivés donnant au moins une pousse résistante à la kanamycine.

| Souches et plasmides binaires | Efficacité de transformation (%) |
|---|---|
| C58'3 (pGA 492-GI) | 0 |
| LBA 4404 (pGA 492-GI) | 4.5 |
| GV 2260 (p35S Gus intron) | 1 |

La coculture de demi-méristèmes avec la souche LBA 4404 (pGA 492-GI) permet d'obtenir un pourcentage de 4,5 % de demi-méristèmes qui initient des pousses transformées résistantes à la kanamycine après 6 semaines dans le milieu M2 contenant 100 mg/l de kanamycine. Ce pourcentage est 4 fois supérieur au pourcentage obtenu avec la souche GV 2260 (p35S Gus intron). Avec la souche C58'3 aucune pousse résistante à la kanamycine n'a été obtenue.

La souche LBA 4404 (pGA 492-GI) a été choisie pour le protocole standard (décrit figure 3) car elle permet d'obtenir de manière routinière des pousses entièrement transformées en nombre élevé.

### Exemple 6 : Influence de la concentration en kanamycine sur la transformation génétique de demi-méristèmes.

Les demi-méristèmes bombardés et cocultivés avec la souche LBA 4404 (pGA 492-GI) sont cultivés pendant 6 semaines en présence de différentes concentrations de kanamycine 50, 100, 200, 400 mg/l dans le milieu M2 (selon le protocole décrit dans l'exemple 3). Après 6 semaines de culture, les pousses vertes résistantes à la kanamycine sont soumises à un test de l'expression de la glucuronidase (protocole décrit dans l'exemple 1, partie 1.5).

Les résultats sont les suivants (Tableau 5) :

| Concentration Kanamycine | Pourcentage de demi-méristèmes donnant des : | | |
|---|---|---|---|
| | pousses axillaires avec des spots GUS+ | pousses portant de larges secteurs GUS+ | pousses axillaires entièrement GUS+ |
| 50 mg/l | 30 | 22.5 | 3.3 |
| 100 mg/l | 30.5 | 18.5 | 3.3 |
| 200 mg/l | 29 | 15.5 | 0 |
| 400 mg/l | 24 | 12 | 0.5 |

Le tableau 5 montre les effets de la kanamycine sur le pourcentage de demi-méristèmes montrant une expression de la glucuronidase. 25 à 30 % des demi-méristèmes bombardés et cocultivés donnent au moins une pousse qui exprime la glucuronidase sous forme de spots quelle que soit la concentration de kanamycine utilisée. Une sélection sur une forte concentration de kanamycine (200 mg/l ou 400 mg/l) diminue légèrement le pourcentage de demi-méristèmes donnant des pousses avec des spots GUS + mais réduit sensiblement le pourcentage de demi-méristèmes donnant des pousses avec de larges secteurs GUS. Seules les concentrations de 50 mg/l et 100 mg/l permettent d'obtenir après sélection 3,3 % de pousses entièrement transformées. La concentration de 50 mg/l de kanamycine a été choisie pour le protocole standard (décrit figures 3 et 4) ; à cette concentration des pousses résistantes et exprimant la glucuronidase peuvent être obtenues de manière routinière et en nombre élevé.

### Exemple 7 : Efficacité de transformation de différents génotypes de tournesol

### 7.1 - Aptitude à la multiplication végétative de demi-méristèmes et à la néoformation de méristèmes foliaires néoformés.

Dans une expérience préliminaire 50 lignées de tournesol ont été sélectionnées pour leur capacité à induire des pousses axillaires à partir de demi-méristèmes et des méristèmes foliaires néoformés sur les feuilles des pousses. Toutes les lignées testées régénèrent des pousses axillaires à partir de demi-méristèmes avec des pourcentages variant de 20 à 90 %. Toutes les lignées testées donnent de 7 à 44 % de demi-méristèmes formant des pousses axillaires qui portent des méristèmes foliaires néoformés à la surface supérieure des feuilles. Plus de 60 % des lignées testées donnent au moins 20 % de demi-méristèmes régénérant des pousses axillaires avec des méristèmes foliaires néoformés sur les feuilles.

### 7.2 - Aptitude à la transformation de demi-méristèmes

Parmi les 50 lignées, 10 présentant la plus forte aptitude à la multiplication végétative de pousses et l'initiation de méristèmes ont été choisies pour évaluer l'efficacité de la transformation génétique de demi-méristèmes.

Environ 200 demi-méristèmes pour chacune des 16 lignées ont été bombardés et cocultivés avec la souche LBA 4404 (pGA 492 GI), puis mis en culture sur le milieu M2 contenant 400 mg/l d'augmentin et 50 mg/l de kanamycine pendant 6 semaines (selon le protocole décrit dans l'exemple 3).

Après 6 semaines de sélection des pousses résistantes à la kanamycine (50 mg/l), toutes les lignées testées donnent au moins un demi-méristème formant des pousses axillaires résistantes à la kanamycine, ce qui montre que le procédé décrit permet d'obtenir des plantes de tournesol entièrement transformées pour 100 % des lignées de tournesol testées (Tableau 6).

L'efficacité de transformation varie de 0,5 à 6 % selon les lignées. Les lignées donnant les plus fortes efficacités de transformation (LG15, LG60 et LG61) avaient aussi les plus fortes aptitudes à la multiplication végétative à partir de demi-méristèmes ce qui suggère une corrélation positive entre l'aptitude à la transformation par le procédé décrit dans l'invention et l'aptitude à la régénération à partir de demi-méristèmes.

| Lignées de tournesol | % de demi-méristèmes donnant des pousses axillaires | % de demi-méristèmes donnant des pousses avec des méristèmes foliaires néoformés | Efficacité de tranformation |
|---|---|---|---|
| HA 300 | 78.5 | 17.5 | 2.5 |
| LG 1 | 65 | 42.5 | 0.5 |
| LG 15 | 85 | 44.5 | 3.4 |
| RHa 356 | 71 | 43.5 | 2 |
| RHa 362 | 46 | 37.5 | 0.5 |
| RHa 274 | 70.5 | 13.5 | 2.6 |
| RHa 297 | 66 | 39.5 | 1.4 |
| RHa 359 | 66 | 39.5 | 2.3 |
| LG 60 | 90 | ND | 3.6 |
| LG 61 | 90 | ND | 6 |

### Exemple 8 : Analyse des plantes transformées régénérées à partir de demi-méristèmes et de leur descendance

Les pousses résistantes à la kanamycine, transférées en système SORBAROD et acclimatées en serre (exemple 3) sont cultivées en serre. Sur ces plantes, plusieurs ramifications se développent avec chacune un capitule.

Des analyses de l'activité glucuronidase ont été faites sur chaque plante et chaque ramification. Pour chaque ramification, un test d'activité glucuronidase est réalisé sur une feuille, sur une fleur ligulée (fleur située au pourtour du capitule possédant un pétale) et sur un fleuron (fleur interne du capitule sans pétale). Les analyses de l'activité GUS ont pour but de déterminer le pourcentage de plantes entièrement transformées qui donneront une descendance dont les gènes insérés (gène NPT II et gène GUS) ségrègeront de façon Mendélienne. De telles plantes doivent être composées de cellules transformées dans tous les tissus et les organes et notamment dans les organes floraux contenant les ovules et le pollen qui donneront après fécondation l'embryon zygotique contenu dans la graine. Une plante est considérée comme étant entièrement transformée si elle possède une activité GUS dans toutes ses ramifications et pour chacune des ramifications, dans les feuilles, les fleurons et les fleurs ligulées.

Le tableau 7 montre que 92 % des plantes transformées cultivées en serre par la technique décrite dans cette invention sont entièrement transformées : pour ces plantes toutes les ramifications possèdent des feuilles, des fleurs ligulées, des fleurons exprimant une activité glucuronidase.

| Plantes T0 entièrement GUS + | Plantes T0 feuilles GUS+ fleurs GUS- | Plantes T0 ramification GUS+ ramification GUS- |
|---|---|---|
| 92 % | 6.5 % | 1.5 % |

Par contre, 8 % des plantes transformées présentent des caractéristiques de plantes chimériques :
- 1,5 % de plantes ont certaines ramifications dans les feuilles et les capitules montrent une activité GUS+ et d'autres ramifications dont les feuilles et les capitules ne montrent pas d'activité glucuronidase.
   Ces plantes chimériques sont issues de pousses dont certains bourgeons axillaires étaient transformés et d'autres bourgeons axillaires n'étaient pas transformés.
- 6,5 % de plantes ont des ramifications dont les feuilles présentent une activité glucuronidase et dont les fleurons ou les fleurs ligulées n'expriment pas la glucuronidase.

Ces plantes chimériques sont issues de pousses dont les bourgeons axillaires composés de cellules transformées au niveau de l'anneau méristématique végétatif à l'origine des parties végétatives (feuilles, tiges et pétioles) et de cellules non transformées au niveau de la partie médullaire du méristème à l'origine des organes floraux et reproducteurs.

Le pourcentage de plantes chimériques obtenu est faible (8 %). Dans la technique décrite dans la présente invention, les plantes ayant une fleur ligulée ou un fleuron n'exprimant pas d'activité glucuronidase sont considérées comme chimériques et sont éliminées.

Les plantes entièrement transformées sont autofécondées par plusieurs passages successifs pour étaler le pollen sur chaque capitule. Les graines sont ensuite récoltées un à deux mois après la fécondation. Les graines sont traitées par 5 heures de trempage dans l'ethrel (solution à 0.1 % dans l'eau), puis décortiquées et stérilisées (selon le protocole décrit dans l'exemple 1). Les graines stériles sont ensuite mises à germer dans le milieu MO contenant 100 mg/l de kanamycine dans les boites de culture Phytatray (SIGMA Réf P1552). Après 15 jours de germination, les plantules transformées résistantes à la kanamycine sont vertes et développent des feuilles vertes. Les plantules non transformées sensibles à la kanamycine sont blanches et ne développent ni racine, ni feuille verte.

Un test d'activité glucuronidase est réalisé sur les feuilles vertes des plantules résistantes à la kanamycine.

Le pourcentage de plantules résistantes à la kanamycine et le pourcentage de plantes exprimant le gène GUS sont déterminés (Tableau 8).

| Lignées T1 | Nombre de graines semées | % de graines résistantes à la kanamycine | % de graines GUS+ |
|---|---|---|---|
| 80-2 | 37 | 78 | 76 |
| 84-1-2 | 40 | 72 | ND |
| 79-1 | 51 | 74 | 20 |
| 107-1 | 8 | 38 | 38 |
| 139-6 | 14 | 35 | 35 |
| 80-1 | 34 | 71 | 71 |
| NT | 40 | 0 | 0 |

Les ségrégations des gènes GUS et NPT II dans les descendances des plantes entièrement transformées suivent une ségrégation de type mendélienne car, dans presque tous les cas, au moins 3/4 des plantes expriment le gène NPT II ou le gène GUS et 1/4 de plantes sont non transformées. Ces résultats confirment que les plantes produites sont bien entièrement transformées et ne sont pas chimériques.

Des analyses moléculaires de ces plantes transgéniques ont été effectuées pour identifier le nombre de loci d'insertions, le nombre de copies de TDNA et pour cartographier les bordures de l'insert.

## Revendications

1. Procédé de production de plantes transgéniques, entièrement transformées en génération T₀, comprenant :
a. une étape de transformation génétique d'un expiant méristématique ;
b. la mise en culture, sur milieu sélectif de l'explant méristématique ayant subi l'étape de transformation ;
c. le prélèvement des bourgeons axillaires transformés et éventuellement des bourgeons foliaires néoformés transformés, obtenus au cours de l'étape b. ;
d. la mise en culture sur milieu sélectif des bourgeons transformés obtenus selon l'étape c. ;
e. la répétition au moins une fois des étapes c. et d. de façon à obtenir des méristèmes secondaires entièrement transformés et éventuellement des méristèmes foliaires néoformés, entièrement transformés ;
f. la régénération, à partir du matériel cellulaire obtenu à l'étape e. de plantes transgéniques.

2. Procédé selon la revendication 1, **caractérisé en ce que**, avant l'étape de transformation, l'explant méristématique est soumis à une étape de préculture d'une durée de 5 à 30 jours, de préférence entre 5 et 8 jours, dans un milieu de culture contenant une cytokinine.

3. Procédé selon la revendication 2. **caractérisé en ce que** l'expiant méristématique consiste en un méristème primaire ou un bourgeon foliaire néoformé ou encore une jeune feuille détachée régénérée à partir d'un méristème primaire ou secondaire et contenant des cellules capables de donner lieu à des méristèmes foliaires néoformés.

4. Procédé selon l'une quelconque des revendications 1 à 3, **caractérisé en ce que** l'étape de transformation comprend le bombardement d'au moins une partie de l'expiant avec des microparticules, suivie par la mise en contact de l'explant bombardé avec Agrobacterium contenant au moins une séquence d'acide nucléique destinée à être introduite dans les cellules méristématiques.

5. Procédé selon l'une quelconque des revendications 1 à 3, **caractérisé en ce que** l'étape de transformation comprend le bombardement d'au moins une partie de l'explant avec des microparticules revêtues d'ADN destiné à être introduit dans les cellules méristématiques.

6. Procédé selon l'une quelconque des revendications 1 à 3, **caractérisé en ce que** l'étape de transformation comprend la mise en contact de l'explant avec une suspension d'Agrobacterium.

7. Procédé selon l'une quelconque des revendications précédentes, **caractérisé en ce que** la séquence d'acide nucléique destinée à être introduite dans les cellules végétales comporte un gène de résistance aux insectes, aux herbicides, aux maladies fongiques, par exemple Sclerotinia sclerotorium ou Botrytis cinerea, un ou plusieurs gènes de protéines de réserve de graines, ou améliorant la qualité des protéines de réserve, des séquences modifiant la maturation du fruit, des séquences conférant une résistance aux virus, un ou plusieurs gènes impliqués dans le métabolisme des acides gras ou des acides aminés ou un gène impliqué dans la stérilité mâle.

8. Procédé selon l'une quelconque des revendications précédentes 4 à 7. **caractérisé en ce que** la séquence d'acide nucléique destinée à être introduite dans les cellules végétales comprend une séquence codant pour un agent permettant la sélection des transformants, par exemple un agent conférant une résistance à un antibiotique tel que la kanamycine.

9. Procédé selon la revendication 4, **caractérisé en ce que** l'Agrobacterium est Agrobacterium tumefaciens, par exemple la souche LBA 4404. contenant un vecteur binaire, par exemple pGA492-GI.

10. Procédé selon l'une quelconque des revendications précédentes, **caractérisé en ce que** le milieu de culture employé lors des étapes de préculture, de coculture et de sélection est le milieu MS additionné de 0.05 à 2.0 mg/l BAP environ, de préférence 0.1 mg/l BAP. et, lorsqu'il s'agit du milieu de sélection, d'au moins un agent sélectif, par exemple la kanamycine, et éventuellement des agents bactériologiques.

11. Procédé selon la revendication 10, **caractérisé en ce que** le milieu est également additionné, pendant les étapes de préculture et de coculture d'un composé phénolique, par exemple l'acétosyringone, capable d'activer les gènes vir d'Agrobacterium.

12. Procédé selon l'une quelconque des revendications précédentes dans lequel la plante transgénique produite est le tournesol transgénique.

13. Procédé selon l'une quelconque des revendications 1 à 11, **caractérisé en ce que** l'explant méristématique provient du coton, d'une espèce végétale oléagineuse, par exemple le tournesol (Helianthus annuus), une espèce appartenant à la famille des légumineuses, par exemple le pois (Pisum sativum), le haricot (Phaseolus vulgaris), une espèce appartenant à la famille des Cucurbitacées, par exemple la courgette (Cucurbita pepo).

14. Procédé selon l'une quelconque des revendications précédentes dans lequel l'explant méristématique provient du tournesol.

15. Procédé de production de méristèmes secondaires entièrement transformés, ou de méristèmes foliaires néoformés entièrement transformés, **caractérisé par** :
i) la mise en oeuvre d'une étape de transformation génétique d'explants méristématiques ;
ii) la mise en culture sur milieu sélectif des explants ayant subi l'étape de transformation ;
iii) le prélèvement des bourgeons axillaires transformés et éventuellement des bourgeons foliaires néoformés transformés obtenus au cours de l'étape ii) ;
iv) la mise en culture, sur milieu sélectif, des bourgeons transformés obtenus selon iii);
v) la répétition, au moins une fois, des étapes iii) et iv).

16. Procédé selon la revendication 15, **caractérisé en ce que** les méristèmes proviennent du tournesol.

## Claims

1. A method for producing transgenic plants wholly transformed in the Tₒ generation, comprising:
a) a step of genetic transformation of a meristematic explant;
b) culturing said transformed meristematic explant on a selection medium;
c) removing said transformed axillary buds detected and optionnaly newly formed leaf buds obtained in step b).
d) culturing said transformed buds obtained in step c) on a selection medium;
e) repeating, at least once, steps c) and d) in order to obtain secondary meristems wholly transformed and optionnaly namely formed leaf meristems wholly transformed;
f) the regeneration, from the cellular material obtained during step e), of transgenic plants.

2. The method according to claim 1 wherein the meristematic explant is precultured for 5 to 30 days, preferably between 5 and 8 days, in a culture medium comprising a cytokine prior to being transformed.

3. Method according to claim 2, **characterized in that** the meristematic explant consists of a primary meristem or a newly formed leaf bud or alternatively a detached young leaf regenerated from a primary or secondary meristem and containing cells capable of giving rise to newly formed leaf meristems.

4. The method according to any one of claims 1 to 3, wherein the transformation step comprises the bombardment at least part of the meristematic explant with microparticles, followed by the bringing of said bombarded explant with *Agrobacterium,* containing at least one nucleic acid sequence intented to be introduced into meristematic cells.

5. Method according to any one of claims 1 to 3, **characterized in that** the transformation step comprises the bombardment of at least part of the explant with microparticles coated with DNA intended to be introduced into the meristematic cells.

6. Method according to any one claims 1 to 3 **characterized in that** the transformation stage comprises the bringing of the explant into contact with a suspension of *Agrobacterium.*

7. Method according to any one of the preceding claims, **characterized in that** the nucleic acid sequence intended to be introduced into plant cells comprises a gene for resistance to insects, to herbicides or to fungal diseases, for example *Sclerotinia sclerotorium or botrytis cinerea,* one or more genes for storage proteins of seeds, or which improve the quality of the storage proteins, sequences which modify the maturation of fruit, sequences which confer resistance to viruses, one or more genes involved in the metabolism of fatty acids or of amino acids or a gene involved in male sterility.

8. Method according to any one of claims 4 to 7, **characterized in that** the nucleic acid sequence intended to be introduced into the plant cells comprises a sequence encoding an agent which allows the selection of transformants, for example an agent which confers resistance to an antibiotic such as kanamycin.

9. Method according to claim 4, **characterized in that** the Agrobacterium is *Agrobacterium tumefaciens,* for example the strain LBA 4404, containing a binary vector, for example pGA492-GI.

10. Method according to any one of the preceding claims, **characterized in that** the culture medium used during the preculture, coculture and selection stages is the MS medium supplemented with about 0.05 to 2.0 mg/l BAP, preferably 0.1 mg/l BAP, and, in the case of the selection medium, with at least one selective agent, for example kanamycin, and optionally bacteriostatic agents.

11. Method according to claim 10, **characterized in that** the medium is also supplemented, during the preculture and coculture stages, with a phenolic compound, for example acetosyringone, which is capable of activating the Agrobacterium *vir* genes.

12. Method according to any one of the preceding claims, wherein the produced transgenic plant is the transgenic sunflower.

13. Method according to any one of claims 1 to 11, **characterized in that** the meristematic explant is obtained from cotton, an oleaginous plant species, for example sunflower (*Helianthus annuus*), a species belonging to the family of leguminous plants, for example pea (*Pisum sativum*), bean (*Phaseolus vulgaris*), a species belonging to the Cucurbitaceae family, for example courgette (*cucurbita pepo*)*.*

14. Method according to any one of the preceding claims, **characterized in that** the meristems are obtained from sunflower.

15. Method of production of wholly transformed secondary meristems, or of wholly transformed newly formed leaf meristems, **characterized by**:
i) the carrying out of a step of genetic transformation of meristematic explants;
ii) the culture, on selective medium, of the explants having undergone the transformation step;
iii) the removal of the transformed axillary buds and optionally of the transformed newly formed leaf buds obtained during step ii);
iv) the culture, on selective medium, of the transformed buds obtained in iii);
v) the repetition, at least once, of steps iii) and iv).

16. Method according to claim 15, **characterized in that** the meristems are obtained from sunflower.

## Patentansprüche

1. Verfahren zur Herstellung transgener Pflanzen, die vollständig in der Generation T₀ transformiert werden, umfassend:
a. einen Schritt zur genetischen Transformation eines meristematischen Explantats;
b. die Kultivierung des meristematischen Explantats, das den Transformationsschritt durchlaufen hat, auf einem Selektionsmedium;
c. die Entnahme der transformierten Achselknospen und gegebenenfalls der transformierten neomorphen Blattknospen, die in Schritt b erhalten wurden;
d. die Kultivierung der gemäß Schritt c) erhaltenen transformierten Knospen auf Selektionsmedium;
e. die wenigstens einmalige Wiederholung der Schritte c) und d), um vollständig transformierte sekundäre Meristeme und gegebenenfalls vollständig transformierte neomorphe Blattmeristeme zu erhalten;
f. die Regeneration von transgenen Pflanzen ausgehend von in Schritt e) erhaltenem Zellmaterial;

2. Verfahren gemäß Anspruch 1, **dadurch gekennzeichnet, dass** das meristematische Explantat vor dem Schritt der Transformation einen Schritt der Vorkultur von 5 bis 30 Tagen Dauer, vorzugsweise zwischen 5 und 8 Tagen, in einem Kulturmedium durchläuft, das ein Cytokinin enthält.

3. Verfahren gemäß Anspruch 2, **dadurch gekennzeichnet, dass** das meristematische Explantat aus einem Primärmeristem oder einer neomorphen Blattknospe oder aber aus einem einzelnen jungen Blatt besteht, das ausgehend von einem primären oder sekundären Meristem regeneriert wurde und Zellen enthält, die die Bildung von neomorphen Blattmeristemen ermöglichen.

4. Verfahren gemäß einem der Ansprüche 1 bis 3, **dadurch gekennzeichnet, dass** der Transformationsschritt das Bombardement mindestens eines Teils des Explantats mit Mikropartikeln umfasst, wobei das bombardierte Explantat anschließend mit Agrobacterium in Kontakt gebracht wird, das mindestens eine Nucleinsäuresequenz enthält, die in die meristematischen Zellen eingeführt werden soll.

5. Verfahren gemäß einem der Ansprüche 1 bis 3, **dadurch gekennzeichnet, dass** der Transformationsschritt das Bombardement mindestens eines Teils des Explantats mit Mikropartikeln umfasst, die mit DNA ummantelt sind, die in die meristematischen Zellen eingeführt werden soll.

6. Verfahren nach einem der Ansprüche 1 bis 3, **dadurch gekennzeichnet, dass** der Transformationsschritt das Inkontaktbringen des Explantats mit einer Agrobacterium-Suspension umfasst.

7. Verfahren nach einem der vorangehenden Ansprüche, **dadurch gekennzeichnet, dass** die Nucleinsäuresequenz, die in die Pflanzenzellen eingeführt werden soll, ein Resistenzgen gegen Insekten, Herbizide, Pilzkrankheiten, zum Beispiel Sclerotinia sclerotorium oder Botrytis cinerea, ein oder mehrere Gene für Speicherproteine von Samen, oder Gene, die die Qualität der Speicherproteine verbessern, die Fruchtreife modifizierende Sequenzen, Virusresistenz vermittelnde Sequenzen, ein oder mehrere am Metabolismus der Fettsäuren oder der Aminosäuren beteiligte Gene oder ein an der maskulinen Sterilität beteiligtes Gen umfasst.

8. Verfahren gemäß einem der vorangehenden Ansprüche 4 bis 7, **dadurch gekennzeichnet, dass** die Nucleinsäuresequenz, die in die Pflanzenzellen eingeführt werden soll, eine Sequenz umfasst, die ein Agens codiert, das die Selektion der Transformanten erlaubt, z.B. ein Agens, das Resistenz gegenüber einem Antibiotikum wie Kanamycin verleiht.

9. Verfahren gemäß Anspruch 4, **dadurch gekennzeichnet, dass** das Agrobacterium Agrobacterium tumefaciens, z.B. Stamm LBA 4404, ist, das einen binären Vektor, z.B. pGA492-GI, enthält.

10. Verfahren gemäß einem der vorangehenden Ansprüche, **dadurch gekennzeichnet, dass** das in den Schritten der Vorkultur, der Co-Kultur und der Selektion verwendete Kulturmedium das Medium MS ist, angereichert mit etwa 0,05 bis 2,0 mg/l BAP, vorzugsweise 0,1 mg/l BAP, und im Fall des Selektionsmediums mit mindestens einem Selektionsagens, z.B. Kanamycin, sowie gegebenenfalls bakteriologischen Agenzien.

11. Verfahren gemäß Anspruch 10, **dadurch gekennzeichnet, dass** das Medium während der Schritte der Vor- und der Co-Kultur, auch mit einer Phenolverbindung, z.B. mit Acetosyringone, angereichert ist, die die Aktivierung der vir-Gene des Agrobacteriums ermöglicht.

12. Verfahren gemäß einem der vorangehenden Ansprüche, wobei die hergestellte transgene Pflanze eine transgene Sonnenblume ist.

13. Verfahren gemäß einem der Ansprüche 1 bis 11, **dadurch gekennzeichnet, dass** das meristematische Explantat von der Baumwolle, von einer Ölpflanzenart, z.B. der Sonnenblume (Helianthus annuus), von einer zur Familie der Leguminosen gehörenden Pflanzenart, z.B. der Erbse (Pisum sativum), der Bohne (Phaseolus vulgaris), von einer zur Familie der Cucurbitaceae gehörenden Pflanzenart, z.B. dem Zuchino (Cucurbita pepo) stammt.

14. Verfahren gemäß einem der vorangehenden Ansprüche, wobei das meristematische Explantat von der Sonnenblume stammt.

15. Verfahren zur Herstellung von vollständig transformierten sekundären Meristemen oder von vollständig transformierten neomorphen Blattmeristemen, **gekennzeichnet durch**:
i) die Ausführung eines Schritts zur genetischen Transformation der meristematischen Explantate;
ii) die Kultivierung der Explantate, die den Transformationsschritt durchlaufen haben, auf einem Selektionsmedium;
iii) die Entnahme der transformierten Achselknospen, sowie gegebenenfalls der transformierten neomorphen Blattknospen, die in Schritt ii) erhalten wurden;
iv) die Kultivierung der gemäß Schritt iii) erhaltenen transformierten Knospen auf einem Selektionsmedium;
v) die mindestens einmalige Wiederholung der Schritte iii) und iv).

16. Verfahren gemäß Anspruch 15, **dadurch gekennzeichnet, dass** die Meristeme von der Sonnenblume stammen.
